# EUROPEAN PATENT SPECIFICATION

(11) **EP 3 716 948 B1**
(45) Date of publication and mention of the grant of the patent: **22.11.2023**
(21) Application number: 18882526.9
(22) Date of filing: 28.08.2018
(51) Int. Cl.: A61K 9/08, A61K 47/40, A61K 38/08, A61K 38/07, A61P 29/00

(54) **NOVEL ANALGESIC PHARMACEUTICAL FORMULATIONS AND USES THEREOF**
NEUARTIGE ANALGETISCHE PHARMAZEUTISCHE FORMULIERUNGEN UND VERWENDUNGEN DAVON
NOUVELLES FORMULATIONS PHARMACEUTIQUES ANALGÉSIQUES ET LEURS UTILISATIONS

(30) Priority: 30.11.2017 US 201762592601 P; 25.04.2018 US 201815962200
(43) Date of publication of application: 07.10.2020
(73) Proprietor: Cytogel Pharma, LLC, Darien, CT 06820 (US)
(72) Inventor: MAIONE, Theodore, E., Green Island, NY 12183 (US); MAGLARIS, Constantine, Basil, New Canaan, CT 06840 (US)
(74) Representative: Engelhard, Markus
(86) International application number: PCT/US2018/048346
(87) International publication number: WO 2019/108285

(56) References cited:
- EP-A1- 1 174 152
- US-A- 5 885 958
- US-A1- 2012 322 740
- US-A1- 2016 264 625
- US-B1- 6 740 639
- DING HUIPING ET AL: "A novel orvinol analog, BU08028, as a safe opioid analgesic without abuse liability in primates", PROCEEDINGS OF THE NATIONAL ACADEMY OF SCIENCES, vol. 113, no. 37, 13 September 2016 (2016-09-13), pages E5511-E5518, XP055830229, US ISSN: 0027-8424, DOI: 10.1073/pnas.1605295113 Retrieved from the Internet: URL:https://www.pnas.org/content/pnas/113/ 37/E5511.full.pdf>
- T. V. KHROYAN ET AL: "The First Universal Opioid Ligand, (2S)-2-[(5R,6R,7R,14S)-N-cyclopropylmethyl -4,5-epoxy-6,14-ethano-3-hydroxy-6-methoxy morphinan-7-yl]-3,3-dimethylpentan-2-ol (BU08028): Characterization of the In Vitro Profile and In Vivo Behavioral Effects in Mouse Models of Acute Pain and Cocaine-Induced Reward", JOURNAL OF PHARMACOLOGY AND EXPERIMENTAL THERAPEUTICS, vol. 336, no. 3, 1 March 2011 (2011-03-01) , pages 952-961, XP055426904, US ISSN: 0022-3565, DOI: 10.1124/jpet.110.175620
- HUANG ET AL: "Agonist treatment did not affect association of mu opioid receptors with lipid rafts and cholesterol reduction had opposite effects on the receptor-mediated signaling in rat brain and CHO cells", BRAIN RESEARCH, ELSEVIER, AMSTERDAM, NL, vol. 1184, 12 October 2007 (2007-10-12), pages 46-56, XP022364558, ISSN: 0006-8993

## Description

### SEQUENCE LISTING

The Sequence Listing for this application is labeled "SeqList-02Apr18-ST25.txt," which was created on April 2, 2018, and is 8 KB.

### BACKGROUND OF THE INVENTION

The major function for opiates is their role in alleviating pain. Other areas where opiates are well-suited for use are conditions relating to gastrointestinal disorders, schizophrenia, obesity, blood pressure, convulsions, and seizures.

Three different opiate receptors have been found: delta (δ), kappa (κ) and mu (µ). Endomorphin-1 peptide (EM-1) and its analogs have been found to exhibit opiate-like activity by binding to the µ opiate receptor.

Activation of the µ receptor is among the most effective means of alleviating a wide range of pain conditions. The unique effectiveness of µ agonists can be attributed to several factors, including their presence in numerous regions of the nervous system that regulate pain processing and activation of multiple mechanisms that limit pain transmission (e.g., inhibiting release of excitatory transmitters from the peripheral nervous system and decreasing cellular excitability in the central nervous system).

Limitations on the use of opioids result from negative side effects, including abuse liability, respiratory depression, and cognitive and motor impairment. Major efforts to develop compounds that maintain analgesic properties while reducing the negative side effects have met with limited success. This is evident from the current epidemic of prescription drug abuse.

Over 100 million patients annually in the United States experience acute or chronic pain and frequently do not achieve adequate relief from existing drugs due to limited efficacy and/or excessive side effects.

Because morphine and other compounds with clinical usefulness act primarily at the µ receptor, pharmaceutical compositions having peptides with high affinity and selectivity for this site are of considerable importance. It would therefore be desirable to have pharmaceutical formulations for delivering these compounds.

Natural endogenous peptides from bovine and human brain that are highly selective for the µ opioid receptor relative to the delta or kappa receptor have been described (see, for example, U.S. Patent No. 6,303,578). These peptides are potent analgesics and have shown promise of reduced abuse liability and respiratory depression.

Ding et al., Proceedings Of The National Academy Of Sciences, vol. 113, no. 37, 2016, relate to an orvinol analog, BU08028, as an opioid analgesic. Khroyan et al., Journal Of Pharmacology And Experimental Therapeutics, vol. 336, no. 3, 2011, pages 952-961, relate to the in vitro profile and in vivo behavioral effects of opioid ligand (2S)-2-[(5R,6R,7R, 14S)-N-cyclopropylmethyl-4,5-epoxy-6, 14-ethano-3 -hydroxy-6-methoxymorphinan-7-yl]-3,3-dimethylpentan-2-ol (BU08028). Huang et al., Brain Research, vol. 1184, 2007, pages 46-56, relate to the effect of an agonist treatment on association of mu opioid receptors with lipid rafts. US 5 885 958 A relates to peptides and linear and cyclic analogs thereof that bind to the mu opiate receptor. US 6 740 639 B1 relates to an inclusion complex, consisting essentially of an opioid peptide of L-tyrosyl-D-alanyl-glycyl-N-methylphenylalanylglycyl-isopropylamide and a cyclodextrin derivative. US 2016/264625 A1 relates to salts of mu-opiate receptor peptides. EP 1174152 A1 relates to an opioid peptide inclusion complex. US 2012/322740 A1 relates to cyclic peptide agonists that bind to the mu opioid receptor.

Cyclized, D-amino acid-containing tetrapeptide analogs of the endomorphins (U.S. Patent No. 5,885,958) have also been described. While these results are promising, the development of improved pharmaceutically-acceptable formulations is needed.

Some currently available opioid formulations are too dilute to meet the needs of patients requiring long term treatment or large drug doses to control pain. For example, sufentanil citrate is currently available in an aqueous solution at a concentration of 50 µg/mL; morphine at 1 µg/mL; morphine sulfate at 20 µg/mL; fentanyl citrate at 20 µg/mL and alfentanil at 500 µg/mL. Simply adding more drug to conventional aqueous formulations is not a viable option to create more concentrated formulations as the opioid compound, especially those that are lipophilic such as fentanyl and its congeners, may precipitate out of solution, which leads to, for example, inconsistent delivery rates, reduced drug absorption, reduced tissue response and clogging of the drug delivery device or other points along the infusion pathway.

The subject invention addresses this need by providing new and advantageous peptide formulations having improved solubility and other properties.

### BRIEF SUMMARY OF THE INVENTION

The invention is set out in the appended set of claims. The subject invention provides compositions for alleviating pain. Specifically, the subject invention provides pharmaceutical formulations of peptides, and/or their salts, having advantageous µ-opioid receptor binding activity.

The peptide is SEQ ID NO: 13: H-Tyr¹-cyclo-(D-Lys²-Trp³-Phe⁴) i.e., amino acids: Tyrosine, Lysine, Tryptophan, and Phenylalanine.

In preferred embodiments, the composition is an aqueous formulation prepared by mixing a salt of the peptide with a cyclodextrin.

The cyclodextrin is 2-Hydroxypropyl-γ-cyclodextrin. The composition comprises a cyclodextrin which is 2-Hydroxypropyl-β-cyclodextrin.

In other embodiments, the peptide may be formulated with, for example, propylene glycol, chremophor, polyethylene glycol and/or polysorbate.

The salt of SEQ ID NO: 13 may be, for example, hydrochloride, maleate, acetate, tartrate, or aspartate salt. The peptide may also be present as a free base.

In a specific embodiment, the formulation contains 20% (w/v) hydroxypropyl-β-cyclodextrin (HPβCD), approximately 0.01 N HCl and 6 mg/mL SEQ ID NO: 13, at a final pH 4.75 - 5.25.

The formulation is a solution.

Formulations of the subject invention can also be used with peptides other than SEQ ID NO: 13 that have µ-opioid receptor binding activity. These peptides may be, for example, any of SEQ ID NO: 1 to 26. The opioid peptide may also be any of those disclosed in U.S. Patent Application Nos. 2012/0322740 A1, US 2013/0178427 A1, US 2015/0315238 A1, US 2016/0009764 A1, US 2016/0176930 A1 or US 2016/0264625 A1.

Another aspect of the subject invention is directed to the compositions for use in a method of treating a patient having a condition that responds to an entity that binds to the µ-opioid receptor. Such a method comprises administering to the patient an effective amount of a pharmaceutical composition of the subject invention. The references to the methods of treatment are to be interpreted as references to compositions of the present invention for use in those methods.

Particular aspects of this method can be performed for the purpose of providing at least one effect selected from (i) analgesia (pain relief), (ii) relief from a gastrointestinal disorder such as diarrhea, (iii) therapy for an opioid drug dependence, (iv) neuropathic pain, and (v) treatment of any condition for which an opioid is indicated.

In some embodiments, the pharmaceutical compositions of the subject invention can be used to treat acute or chronic pain. Uses for the compositions also include, but are not be limited to, use as antimigraine agents, immunomodulatory agents, immunosuppressive agents and/or anti arthritic agents.

Certain aspects of the methods of the present disclosure, such as treatment of pain or opioid drug dependence, are directed to patients having a history of opioid substance abuse.

In certain aspects of the present methods, the composition of the subject invention is administered parenterally. The administration may be done by, for example, intravenous, intramuscular, or subcutaneous administration. In other embodiments, the composition is administered orally.

### BRIEF DESCRIPTION OF THE SEQUENCES

SEQ ID NO: 1 is a peptide useful according to the subject invention.
SEQ ID NO:2 is a peptide useful according to the subject invention.
SEQ ID NO:3 is a peptide useful according to the subject invention.
SEQ ID NO:4 is a peptide useful according to the subject invention.
SEQ ID NO:5 is a peptide useful according to the subject invention.
SEQ ID NO:6 is a peptide useful according to the subject invention.
SEQ ID NO:7 is a peptide useful according to the subject invention.
SEQ ID NO:8 is a peptide useful according to the subject invention.
SEQ ID NO:9 is a peptide useful according to the subject invention.
SEQ ID NO: 10 is a peptide useful according to the subject invention.
SEQ ID NO: 11 is a peptide useful according to the subject invention.
SEQ ID NO: 12 is a peptide useful according to the subject invention.
SEQ ID NOS: 13-26 are additional peptides useful according to the subject invention.

### DETAILED DESCRIPTION OF THE INVENTION

The invention is set out in the appended set of claims. The subject invention provides compositions for alleviating pain. Specifically, the subject invention provides pharmaceutical formulations of peptides, and/or their salts, having advantageous µ-opioid receptor binding activity. Advantageously, the concentration of the peptide in the formulation of the subject invention can be at or above 4, 5, 6, 7, 8, 9, or even 10 mg/ml. In preferred embodiments, the formulation is a solution.

The peptide is SEQ ID NO: 13: H-Tyr¹-cyclo-(D-Lys²-Trp³-Phe⁴) i.e., amino acids: Tyrosine, Lysine, Tryptophan, and Phenylalanine.

The composition is an aqueous formulation of the peptide with a cyclodextrin.

The cyclodextrin is 2-Hydroxypropyl-β-cyclodextrin. The concentration of cyclodextrin is from 15% to 25%.

The composition can be formed by, for example, combining a salt of the peptide with the cyclodextrin in an aqueous solution.

The salt of the peptide may be, for example, hydrochloride, maleate, acetate, tartrate, or aspartate salt. The peptide may also be present as the free base.

In a specific embodiment the formulation contains 20% (w/v) hydroxypropyl-β-cyclodextrin (HPβCD), approximately 0.01 N HCl and 6 mg/mL SEQ ID NO: 13, at a final pH 4.75 - 5.25.

In other embodiments, the peptide may be formulated with, for example, propylene glycol, chremophor, polyethylene glycol and/or polysorbate.

The formulation is a solution.

Formulations of the subject invention can also be used with opioid peptides other than SEQ ID NO: 13. These opioid peptides may be, for example, any of SEQ ID NO: 1 to 26. The opioid peptide may also be any of those disclosed in U.S. Patent Application Nos. 2012/0322740 A1, US 2013/0178427 A1, US 2015/0315238 A1, US 2016/0009764 A1, US 2016/0176930 A1 or US 2016/0264625 A1.

Peptides that can be used according to the subject invention include those having the general formula Tyr-X₁-X₂-X₃ wherein X₁ is Pro, D-Lys or D-Orn; X₂ is Trp, Phe or N-alkyl-Phe wherein alkyl contains 1 to about 6 carbon atoms; and X₃ is Phe, Phe-NH₂, D-Phe, D-Phe-NH₂ or p-Y-Phe wherein Y is NO₂, F, Cl or Br. Some specific peptides of the disclosure are:
H-Tyr-Pro-Trp-Phe-NH₂ (SEQ ID NO:1)
H-Tyr-Pro-Phe-Phe-NH₂ (SEQ ID NO:2)
H-Tyr-Pro-Trp-Phe-OH (SEQ ID NO:3)
H-Tyr-Pro-Phe-Phe-OH (SEQ ID NO:4)
H-Tyr-Pro-Trp-D-Phe-NH₂ (SEQ ID NO:5)
H-Tyr-Pro-Phe-D-Phe-NH₂ (SEQ ID NO:6)
H-Tyr-Pro-Trp-pNO₂ -Phe-NH₂ (SEQ ID NO:7)
H-Tyr-Pro-Phe-pNO₂ -Phe-NH₂ (SEQ ID NO:8)
H-Tyr-Pro-N-Me-Phe-Phe-NH₂ (SEQ ID NO:9)
H-Tyr-Pro-N-Et-Phe-Phe-NH₂ (SEQ ID NO:10)
H-Tyr-Pro-N-Me-Phe-D-Phe-NH₂ (SEQ ID NO:11)
H-Tyr-Pro-N-Et-Phe-D-Phe-NH₂ (SEQ ID NO:12)
H-Tyr-c-[D-Lys-Trp-Phe] (SEQ ID NO:13)
H-Tyr-c-[D-Lys-Phe-Phe] (SEQ ID NO:14)
H-Tyr-c-[D-Orn-Trp-Phe] (SEQ ID NO:15)
H-Tyr-c-[D-Orn-Phe-Phe] (SEQ ID NO:16)
H-Tyr-c-[D-Lys-Trp-pNO₂ -Phe] (SEQ ID NO:17)
H-Tyr-c-[D-Lys-Phe-pNO₂ -Phe] (SEQ ID NO:18)
H-Tyr-c-[D-Orn-Trp-pNO₂ -Phe] (SEQ ID NO:19)
H-Tyr-c-[D-Orn-Phe-pNO₂ -Phe] (SEQ ID NO:20)
H-Tyr-c-[D-Lys-N-Me-Phe-Phe] (SEQ ID NO:21)
H-Tyr-c-[D-Orn-N-Me-Phe-Phe] (SEQ ID NO:22)
H-Tyr-c-[D-Lys-N-Et-Phe-Phe] (SEQ ID NO:23)
H-Tyr-c-[D-Orn-N-Et-Phe-Phe] (SEQ ID NO:24)
H-Tyr-c-[D-Lys-N-Me-Phe-D-Phe] (SEQ ID NO:25)
H-Tyr-c-[D-Lys-N-Et-Phe-D-Phe] (SEQ ID NO:26)

The last fourteen peptides listed are cyclic peptides whose linear primary amino acid sequences are given in SEQ ID NO: 13 through SEQ ID NO: 26. In this context, the applicants relate to U.S. Patent No. 6,303,578.

Another aspect of the invention is directed to the compositions described herein for use in a method of treating a patient having a condition that responds to an entity that binds to the µ-opioid receptor. Such a method comprises administering to the patient an effective amount of a pharmaceutical composition of the subject invention. The references to the methods of treatment in this description are to be interpreted as references to compositions of the present invention for use in those methods.

Particular aspects of this method can be followed for the purpose of providing at least one effect selected from (i) analgesia (pain relief), (ii) relief from a gastrointestinal disorder such as diarrhea, (iii) therapy for an opioid drug dependence, (iv) neuropathic pain, and (v) treatment of any condition for which an opioid is indicated.

In some embodiments the pharmaceutical compositions of the subject invention can be used to treat acute or chronic pain. Uses for the compositions also include, but are not be limited to, use as antimigraine agents, immunomodulatory agents, immunosuppressive agents and/or anti arthritic agents.

Certain aspects of the methods of the present disclosure, such as treatment of pain or opioid drug dependence, are directed to patients having a history of opioid substance abuse.

In certain aspects of the present methods, the composition of the subject invention is administered parenterally. The administration may be done by, for example, intravenous, intramuscular, or subcutaneous administration. In other embodiments, the composition is administered orally.

The pharmaceutical compositions can be delivered in any suitable dosage form, such as, for example, a liquid, gel, solid, cream, or paste dosage form. In one embodiment, the compositions can be adapted to give sustained release of the peptide.

The pharmaceutical compositions include, but are not limited to, those forms suitable for oral, rectal, nasal, topical, (including buccal and sublingual), transdermal, vaginal, parenteral (including intramuscular, subcutaneous, and intravenous), spinal (epidural, intrathecal), and central (intracerebroventricular) administration. The compositions can, where appropriate, be conveniently provided in discrete dosage units.

### Selected Definitions

"Pharmaceutically acceptable" means approved or approvable by a regulatory agency of the Federal or a state government or the corresponding agency in countries other than the United States, or that is listed in the U.S. Pharmacopoeia or other generally recognized pharmacopoeia for use in animals, and more particularly, in humans.

"Pharmaceutically acceptable salt" refers to a salt of a compound of the disclosure that is pharmaceutically acceptable and that possesses the desired pharmacological activity of the parent compound. In particular, such salts are non-toxic may be inorganic or organic acid addition salts and base addition salts. Specifically, such salts include: (1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl)benzoic acid, cinnamic acid, mandelic acid, methanesulfonic acid, ethanesulfonic acid, 1,2-ethane-di sulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2.2.2]-oct-2-ene-1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; or (2) salts formed when an acidic proton present in the parent compound either is replaced by a metal ion, e.g., an alkali metal ion, an alkaline earth ion, or an aluminum ion; or coordinates with an organic base such as ethanolamine, diethanolamine, triethanolamine, N-methylglucamine and the like. Salts further include, by way of example only, sodium, potassium, calcium, magnesium, ammonium, tetraalkylammonium, and the like; and when the compound contains a basic functionality, salts of non-toxic organic or inorganic acids, such as hydrochloride, hydrobromide, tartrate, mesylate, acetate, maleate, oxalate and the like.

"Pharmaceutically acceptable vehicle" refers to a diluent, adjuvant, excipient or carrier with which a compound of the disclosure is administered. A "pharmaceutically acceptable excipient" refers to a substance that is non-toxic, biologically tolerable, and otherwise biologically suitable for administration to a subject, such as an inert substance, added to a pharmacological composition or otherwise used as a vehicle, carrier, or diluent to facilitate administration of a agent and that is compatible therewith. Examples of excipients include calcium carbonate, calcium phosphate, various sugars and types of starch, cellulose derivatives, gelatin, vegetable oils, and polyethylene glycols.

"Subject" includes humans. The terms "human," "patient," and "subject" are used interchangeably herein.

"Treating" or "treatment" of any disease or disorder refers, in one embodiment, to ameliorating the disease or disorder (i.e., arresting or reducing the development of the disease or at least one of the clinical symptoms thereof). In another embodiment "treating" or "treatment" refers to ameliorating at least one physical parameter, which may not be discernible by the subject. In yet another embodiment, "treating" or "treatment" refers to modulating the disease or disorder, either physically, (e.g., stabilization of a discernible symptom), physiologically, (e.g., stabilization of a physical parameter), or both. In yet another embodiment, "treating" or "treatment" refers to delaying the onset of the disease or disorder.

As used herein, the terms "reducing," "inhibiting," "blocking," "preventing", alleviating," or "relieving" when referring to a compound (e.g., a peptide), mean that the compound brings down the occurrence, severity, size, volume, or associated symptoms of a condition, event, or activity by at least about 7.5%, 10%, 12.5%, 15%, 17.5%, 20%, 22.5%, 25%, 27.5%, 30%, 35%, 40%, 45%, 50%, 55%, 60%, 65%, 70%, 75%, 80%, 90%, or 100% compared to how the condition, event, or activity would normally exist without application of the compound or a composition comprising the compound. The terms "increasing," "elevating," "enhancing," "upregulating," "improving," or "activating" when referring to a compound mean that the compound increases the occurrence or activity of a condition, event, or activity by at least about 7.5%, 10%, 12.5%, 15%, 17.5%, 20%, 22.5%, 25%, 27.5%, 30%, 35%, 40%, 45%, 50%, 55%, 60%, 65%, 70%, 75%, 80%, 90%, 100%, 150%, 200%, 250%, 300%, 400%, 500%, 750%, or 1000% compared to how the condition, event, or activity would normally exist without application of the compound or a composition comprising the compound.

The term "modulating" includes "reducing," "inhibiting," "blocking," "preventing", alleviating," "relieving," "increasing," "elevating," "enhancing," "upregulating," "improving," and "activating."

In treatment methods according to the disclosure, a therapeutically effective amount of a pharmaceutical agent according to the disclosure is administered to a subject suffering from or diagnosed as having such a disease, disorder, or condition. A "therapeutically effective amount" means an amount or dose sufficient to generally bring about the desired therapeutic or prophylactic benefit in patients in need of such treatment for the designated disease, disorder, or condition.

Effective amounts or doses of the compounds of the present disclosure may be ascertained by routine methods such as modeling, dose escalation studies or clinical trials, and by taking into consideration routine factors, e.g., the mode or route of administration or drug delivery, the pharmacokinetics of the compound, the severity and course of the disease, disorder, or condition, the subject's previous or ongoing therapy, the subject's health status and response to drugs, and the judgment of the treating physician. An example of a dose is in the range of from about 0.001 to about 200 mg of compound per kg of subject's body weight per day, preferably about 0.05 to 100 mg/kg/day, or about 1 to 35 mg/kg/day, in single or divided dosage units (e.g., BID, TID, QID). For a 70-kg human, an illustrative range for a suitable dosage amount is from about 0.05 to about 7 g/day, or about 0.2 to about 2.5 g/day.

"Compounds of the present disclosure," and equivalent expressions, are meant to embrace the exemplified compounds as described herein, which expression includes the pharmaceutically acceptable salts, and solvates, e.g., hydrates, where the context so permits.

Such compounds can be formulated as pharmaceutical compositions and administered to a subject in need of treatment, for example a mammal, such as a human patient, in a variety of forms adapted to the chosen route of administration. For example compounds of the disclosure may be formulated for administration, orally, nasally, intraperitoneally, or parenterally, by intravenous, intramuscular, topical, or subcutaneous routes, or by injection into tissue.

### Routes of Administration and Dosage Forms

In certain embodiments, the compounds may be administered intravenously or intraperitoneally by infusion or injection. Solutions of the compounds can be prepared in water, optionally mixed with a nontoxic surfactant. Under ordinary conditions of storage and use, these preparations can contain a preservative to prevent the growth of microorganisms.

The pharmaceutical dosage forms suitable for injection or infusion can include sterile aqueous solutions or dispersions or sterile powders comprising the compounds that are adapted for the extemporaneous preparation of sterile injectable or infusible solutions or dispersions, optionally encapsulated in liposomes. Preferably, the ultimate dosage form should be sterile, fluid, and stable under the conditions of manufacture and storage. The liquid carrier or vehicle can be a solvent or liquid dispersion medium comprising, for example, water, ethanol, a polyol (for example, glycerol, propylene glycol, liquid polyethylene glycols, and the like), vegetable oils, nontoxic glyceryl esters, and suitable mixtures thereof. The proper fluidity can be maintained by, for example, the formation of liposomes, by the maintenance of the required particle size in the case of dispersions, or by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars, buffers, or sodium chloride. Prolonged absorption of the injectable compositions can be brought about by the use in the compositions of agents delaying absorption, for example, aluminum monostearate and gelatin.

Sterile injectable solutions are prepared by incorporating the compounds in the required amount in the appropriate solvent as described herein with various of the other ingredients enumerated herein, as required, preferably followed by filter sterilization. In the case of sterile powders for the preparation of sterile injectable solutions, the preferred methods of preparation are vacuum drying and freeze drying techniques, which yield a powder of the active ingredient plus any additional desired ingredient present in the previously sterile-filtered solutions.

The compositions of the subject invention may also be administered orally, in combination with a pharmaceutically acceptable vehicle such as an inert diluent or an assimilable edible carrier. They may be enclosed in hard or soft shell gelatin capsules, may be compressed into tablets, or may be incorporated directly with the food of the patient's diet.

For oral therapeutic administration, the compounds may be combined with one or more excipients and used in the form of ingestible tablets, buccal tablets, troches, capsules, elixirs, suspensions, syrups, wafers, and the like. Such compositions and preparations should contain at least 0.1% of a compound of the present disclosure. The percentage of the compound of the disclosure present in such compositions and preparations may, of course, be varied and may conveniently be between about 2% to about 60% of the weight of a given unit dosage form. The amount of the compound in such therapeutically useful compositions is such that an effective dosage level will be obtained.

The tablets, troches, pills, capsules, and the like may also contain one or more of the following: binders such as gum tragacanth, acacia, corn starch or gelatin; excipients such as dicalcium phosphate; a disintegrating agent such as corn starch, potato starch, alginic acid, and the like; a lubricant such as magnesium stearate; and a sweetening agent such as sucrose, fructose, lactose, or aspartame, or a flavoring agent such as peppermint, oil of wintergreen, or cherry flavoring may be added.

When the unit dosage form is a capsule, it may contain, in addition to materials of the above type, a liquid carrier, such as a vegetable oil or a polyethylene glycol.

Various other materials may be present as coatings or for otherwise modifying the physical form of the solid unit dosage form. For instance, tablets, pills, or capsules may be coated with gelatin, wax, shellac, or sugar, and the like. A syrup or elixir may contain the active compound, sucrose or fructose as a sweetening agent, methyl and propylparabens as preservatives, a dye, and flavoring such as cherry or orange flavor.

Of course, any material used in preparing any unit dosage form should be pharmaceutically acceptable and substantially non-toxic in the amounts employed.

In addition, the compounds may be incorporated into sustained-release preparations and devices. For example, the compounds may be incorporated into time release capsules, time release tablets, time release pills, and time release polymers or nanoparticles.

Pharmaceutical compositions for topical administration of the peptides to the epidermis (mucosal or cutaneous surfaces) can be formulated as ointments, creams, lotions, gels, or as a transdermal patch. Such transdermal patches can contain penetration enhancers such as linalool, carvacrol, thymol, citral, menthol, t-anethole, and the like. Ointments and creams can, for example, include an aqueous or oily base with the addition of suitable thickening agents, gelling agents, colorants, and the like. Lotions and creams can include an aqueous or oily base and typically also contain one or more emulsifying agents, stabilizing agents, dispersing agents, suspending agents, thickening agents, coloring agents, and the like. Gels preferably include an aqueous carrier base and include a gelling agent such as crosslinked polyacrylic acid polymer, a derivatized polysaccharide (e.g., carboxymethyl cellulose), and the like.

Pharmaceutical compositions suitable for topical administration in the mouth (e.g., buccal or sublingual administration) include lozenges comprising the composition in a flavored base, such as sucrose, acacia, or tragacanth; pastilles comprising the composition in an inert base such as gelatin and glycerin or sucrose and acacia; and mouthwashes comprising the active ingredient in a suitable liquid carrier. The pharmaceutical compositions for topical administration in the mouth can include penetration enhancing agents, if desired.

Useful solid carriers include finely divided solids such as talc, clay, microcrystalline cellulose, silica, alumina, and the like. Other solid carriers include nontoxic polymeric nanoparticles or microparticles. Useful liquid carriers include water, alcohols, or glycols, or water/alcohol/glycol blends, in which the compounds can be dissolved or dispersed at effective levels, optionally with the aid of non-toxic surfactants. Adjuvants such as fragrances and additional antimicrobial agents can be added to optimize the properties for a given use. The resultant liquid compositions can be applied from absorbent pads, used to impregnate bandages and other dressings, or sprayed onto the affected area using pump-type or aerosol sprayers.

Thickeners such as synthetic polymers, fatty acids, fatty acid salts and esters, fatty alcohols, modified celluloses, or modified mineral materials can also be employed with liquid carriers to form spreadable pastes, gels, ointments, soaps, and the like, for application directly to the skin of the user.

Examples of useful dermatological compositions which can be used to deliver the compounds to the skin are known to the art; for example, see Jacquet et al. (U.S. Patent No. 4,608,392), Geria (U.S. Patent No. 4,992,478), Smith et al. (U.S. Patent No. 4,559,157) and Wortzman (U.S. Patent No. 4,820,508).

The concentration of the therapeutic compounds of the disclosure in such formulations can vary widely depending on the nature of the formulation and intended route of administration. For example, the concentration of the compounds in a liquid composition, such as a lotion, can preferably be from about 0.1-25% by weight, or, more preferably, from about 0.5-10% by weight. The concentration in a semi-solid or solid composition such as a gel or a powder can preferably be about 0.1-5% by weight, or, more preferably, about 0.5-2.5% by weight.

Pharmaceutical compositions for spinal administration or injection into amniotic fluid can be provided in unit dose form in ampoules, pre-filled syringes, small volume infusion, or in multi-dose containers, and can include an added preservative. The compositions for parenteral administration can be suspensions, solutions, or emulsions, and can contain excipients such as suspending agents, stabilizing agents, and dispersing agents.

A pharmaceutical composition suitable for rectal administration comprises a peptide of the present disclosure in combination with a solid or semisolid (e.g., cream or paste) carrier or vehicle. For example, such rectal compositions can be provided as unit dose suppositories. Suitable carriers or vehicles include cocoa butter and other materials commonly used in the art.

According to one embodiment, pharmaceutical compositions of the present invention suitable for vaginal administration are provided as pessaries, tampons, creams, gels, pastes, foams, or sprays containing a peptide of the disclosure in combination with carriers as are known in the art. Alternatively, compositions suitable for vaginal administration can be delivered in a liquid or solid dosage form.

Pharmaceutical compositions suitable for intra-nasal administration are also encompassed by the present invention. Such intra-nasal compositions comprise a peptide of the disclosure in a vehicle and suitable administration device to deliver a liquid spray, dispersible powder, or drops. Drops may be formulated with an aqueous or non-aqueous base also comprising one or more dispersing agents, solubilizing agents, or suspending agents. Liquid sprays are conveniently delivered from a pressurized pack, an insufflator, a nebulizer, or other convenient means of delivering an aerosol comprising the peptide. Pressurized packs comprise a suitable propellant such as dichlorodifluoromethane, trichlorofluoromethane, dichlorotetrafluoroethane, carbon dioxide, or other suitable gas as is well known in the art. Aerosol dosages can be controlled by providing a valve to deliver a metered amount of the peptide.

The compounds may be combined with an inert powdered carrier and inhaled by the subject or insufflated.

The exact amount (effective dose) of the agent will vary from subject to subject, depending on, for example, the species, age, weight, and general or clinical condition of the subject, the severity or mechanism of any disorder being treated, the particular agent or vehicle used, the method and scheduling of administration, and the like. A therapeutically effective dose can be determined empirically, by conventional procedures known to those of skill in the art. See, e.g., The Pharmacological Basis of Therapeutics, Goodman and Gilman, eds., Macmillan Publishing Co., New York. For example, an effective dose can be estimated initially either in cell culture assays or in suitable animal models. The animal model may also be used to determine the appropriate concentration ranges and routes of administration. Such information can then be used to determine useful doses and routes for administration in humans. Methods for the extrapolation of effective dosages in mice and other animals to humans are known to the art; for example, see U.S. Patent No. 4,938,949. A therapeutic dose can also be selected by analogy to dosages for comparable therapeutic agents.

The particular mode of administration and the dosage regimen will be selected by the attending clinician, taking into account the particulars of the case (e.g., the subject, the disease, the disease state involved, and whether the treatment is prophylactic). Treatment may involve daily or multi-daily doses of compound(s) over a period of a few days to months, or even years.

In general, however, a suitable dose will be in the range of from about 0.001 to about 100 mg/kg of body weight per day, preferably from about 0.01 to about 100 mg/kg of body weight per day, more preferably, from about 0.1 to about 50 mg/kg of body weight per day, or even more preferred, in a range of from about 1 to about 10 mg/kg of body weight per day. For example, a suitable dose may be about 1 mg/kg, 10 mg/kg, or 50 mg/kg of body weight per day.

The compounds can be conveniently administered in unit dosage form, containing for example, about 0.05 to about 10000 mg, about 0.5 to about 10000 mg, about 5 to about 1000 mg, or about 50 to about 500 mg of active ingredient per unit dosage form.

The compounds can be administered to achieve peak plasma concentrations of, for example, from about 0.25 to about 200 µM, about 0.5 to about 75 µM, about 1 to about 50 µM, about 2 to about 30 µM, or about 5 to about 25 µM. Exemplary desirable plasma concentrations include at least 0.25, 0.5, 1, 5, 10, 25, 50, 75, 100 or 200 µM. For example, plasma levels may be from about 1 to about 100 micromolar or from about 10 to about 25 micromolar. This may be achieved, for example, by the intravenous injection of a 0.05 to 5% solution of the compounds, optionally in saline, or orally administered as a bolus containing about 1 to about 100 mg of the compounds. Desirable blood levels may be maintained by continuous or intermittent infusion.

SEQ ID NO: 13 will be included in the compositions within a therapeutically useful and effective concentration range, as determined by routine methods that are well known in the medical and pharmaceutical arts. For example, a typical composition can include one or more of the peptide at a concentration in the range of at least about 4 mg/ml, more preferably at least 5 mg/ml and most preferably at least 6 mg/ml.

The compounds may conveniently be presented in a single dose or as divided doses administered at appropriate intervals, for example, as one dose per day or as two, three, four or more sub-doses per day. The sub-dose itself may be further divided, e.g., into a number of discrete loosely spaced administrations; such as multiple inhalations from an insufflator.

Optionally, the pharmaceutical compositions of the present invention can include one or more other therapeutic agents, e.g., as a combination therapy. The additional therapeutic agent(s) will be included in the compositions within a therapeutically useful and effective concentration range, as determined by routine methods that are well known in the medical and pharmaceutical arts. The concentration of any particular additional therapeutic agent may be in the same range as is typical for use of that agent as a monotherapy, or the concentration may be lower than a typical monotherapy concentration if there is a synergy when combined with a peptide of the present disclosure.

### Methods of Treatment

The present invention provides for the compositions of the subject invention for use in the treatment of conditions that can be improved through binding at the µ-opioid receptor. This can include, for example, pain, discomfort associated with gastrointestinal disorders, and treatment of drug dependence. The references to the methods of treatment in this description are to be interpreted as references to the compositions of the present invention for use in those methods.

Methods for providing analgesia (alleviating or reducing pain), relief from gastrointestinal disorders such as diarrhea, and therapy for drug dependence in patients, such as mammals, including humans, comprise administering to a patient suffering from one of the aforementioned conditions an effective amount of the composition of the subject invention.

Diarrhea may be caused by a number of sources, such as infectious disease, cholera, or an effect or side-effect of various drugs or therapies, including those used for cancer therapy. Preferably, the peptide is administered parenterally or enterally. The dosage of the effective amount of the peptides can vary depending upon the age and condition of each individual patient to be treated. However, suitable unit dosages typically range from about 0.01 to about 100 mg. For example, a unit dose can be in the range of about 0.2 mg to about 50 mg. Such a unit dose can be administered more than once a day, e.g., two or three times a day.

The following examples are included to demonstrate certain aspects of the invention. It should be appreciated by those of skill in the art that the techniques disclosed in the examples, which represent techniques known to function well in practicing the invention, can be considered to constitute preferred modes for its practice; however, those of skill in the art should, in light of the present disclosure, appreciate that many changes can be made in the specific disclosed embodiments and still obtain a like or similar result without departing from the scope of the invention. The examples, thus, are provided for illustration purposes only and are not intended to be limiting. The invention is defined in the appended set of claims.

### EXAMPLE 1: PREPARATION OF A SEQ ID NO: 13 COMPOSITION OF THE SUBJECT INVENTION

A formulation is prepared that includes SEQ ID NO: 13, 20% HPβCD, and approximately 0.01N HCl made in sterile water by the following method.

First, the HPβCD and HCl are added to sterile water. SEQ ID NO: 13 is added at 7.5 mg/mL. The mixture is stirred overnight at room temperature and then filtered through 0.45 µm filter to remove any insoluble material.

The in-process concentration is determined by HPLC analysis.

The filtered solution is then diluted (with 20% HPβCD solution without HCl) to 6 mg/mL SEQ ID NO: 13 after analysis and filtered again through 0.22 µm filters.

### EXAMPLE 2: INGREDIENTS OF A SEQ ID NO: 13 COMPOSITION OF THE SUBJECT INVENTION

Table 1 shows the ingredients of a preferred embodiment of the composition of the subject invention.

| **Table 1** | | |
|---|---|---|
| **Component** | **Quantity per mL** | **%** |
| SEQ ID NO: 13⁺ | 6.1 mg | 0.61% (w/v) |
| HCl USP | 0.365 mg* (10 µmol) | 0.036% (w/v)* (10 mM) |
| HPβCD, USP | 200 mg | 20% (w/v) |
| Water for Injection, USP/EP | To 1.0 mL | To 100% |

| | | |
|---|---|---|
| ⁺ Corrected for Peptide and Purity Content Peptide Content = 97.4% Purity (HPLC) = 99.7% * maximum HCl level based on final pH 4.75-5.25 | | |

### EXAMPLE 3: ADDITIONAL FORMULATIONS

| Additional SEQ ID NO: 13 formulations showing antinococeptive activity in the rat tail flick assay | |
|---|---|
| 20% 2-Hydroxypropyl-B-cyclodextrin | |
| 20% 2-Hydroxypropyl-B-cyclodextrin | 0.01N HCl |
| 40% Propylene Glycol | 0.01N HCl |
| 40% Propylene Glycol | 0.02N Acetic Acid |
| 40% Propylene Glycol | 0.02N Na Citrate, pH 3 |
| 20% PEG-300 | 0.02N Acetic Acid |
| 10% Chremophor RH60 | 0.01N HCl |
| 10% Chremophor RH60 | 0.02N Acetic |
| 10% Chremophor EL | 0.01N HCl |
| 10% Chremophor EL | 0.02N Acetic |
| 10% Polysorbate 80 | 0.01N HCl |
| 10% Polysorbate 80 | 0.02N Acetic |

## Claims

1. A composition having µ-opioid receptor binding activity wherein the composition comprises a µ-opioid receptor binding peptide and a cyclodextrin, wherein the composition is an aqueous solution, wherein the peptide is SEQ ID NO: 13 at a concentration of at least 4 mg/ml, and
wherein the cyclodextrin is 2-Hydroxypropyl-β-cyclodextrin (HPβC) at a concentration from 15% to 25%.

2. The composition, according to claim 1, having a pH from 4.75 - 5.25, and/or comprising about 0.01 N HCl.

3. The composition, according to claim 1, wherein the concentration of SEQ ID NO: 13 is at least 6 mg/ml, the concentration of HPβC is about 20% and the composition is a solution, wherein, more preferably, the composition comprising SEQ ID NO: 13 as peptide further comprises acetate ions.

4. An *in vitro* method for modulating the activity of a µ-opioid receptor wherein said method comprises contacting said receptor with a composition of claim 1.

5. A composition according to any of claims 1 - 3, for use in a method for alleviating pain that can be treated by modulation of the µ-opioid receptor, wherein said method comprises administering, to a subject in need of such treatment, an effective amount of a composition of claim 1.

## Patentansprüche

1. Zusammensetzung mit µ-Opioidrezeptor-Bindungsaktivität, wobei die Zusammensetzung ein µ-Opioidrezeptor-bindendes Peptid und ein Cyclodextrin umfasst, wobei die Zusammensetzung eine wässrige Lösung ist, wobei das Peptid SEQ ID NO: 13 in einer Konzentration von mindestens 4 mg/ml ist, und
wobei das Cyclodextrin 2-Hydroxypropyl-β-cyclodextrin (HPβC) in einer Konzentration von 15% bis 25% ist.

2. Zusammensetzung nach Anspruch 1, mit einem pH-Wert von 4,75 - 5,25, und/oder umfassend etwa 0,01 N HCl.

3. Zusammensetzung nach Anspruch 1, wobei die Konzentration von SEQ ID NO: 13 mindestens 6 mg/ml beträgt, die Konzentration von HPβC etwa 20% beträgt und die Zusammensetzung eine Lösung ist, wobei, bevorzugter, die Zusammensetzung, die SEQ ID NO: 13 als Peptid umfasst, ferner Acetat-Ionen umfasst.

4. *In vitro*-Verfahren zum Modulieren der Aktivität eines µ-Opioidrezeptors, wobei das Verfahren das In-Kontakt-Bringen des Rezeptors mit einer Zusammensetzung nach Anspruch 1 umfasst.

5. Zusammensetzung nach einem der Ansprüche 1-3 zur Verwendung in einem Verfahren zur Linderung von Schmerzen, die durch Modulation des µ-Opioidrezeptors behandelt werden können, wobei das Verfahren das Verabreichen einer wirksamen Menge einer Zusammensetzung nach Anspruch 1 an ein Subjekt, das einer solchen Behandlung bedarf, umfasst.

## Revendications

1. Composition présentant une activité de liaison au récepteur opioïde µ, dans laquelle la composition comprend un peptide de liaison au récepteur opioïde µ et une cyclodextrine, dans laquelle la composition est une solution aqueuse, dans laquelle le peptide est SEQ ID NO: 13 à une concentration d'au moins 4 mg/ml, et
dans laquelle la cyclodextrine est la 2-hydroxypropyl-β-cyclodextrine (HPβC) à une concentration comprise entre 15 % et 25 %.

2. Composition selon la revendication 1, possédant un pH compris entre 4,75 et 5,25, et/ou comprenant environ 0,01 N de HCl.

3. Composition selon la revendication 1, dans laquelle la concentration de SEQ ID NO: 13 est au moins 6 mg/ml, la concentration de l'HPβC est environ 20 % et la composition est une solution, dans laquelle, de manière davantage préférée, la composition comprenant SEQ ID NO: 13 en tant que peptide comprend en outre des ions acétate.

4. Procédé *in vitro* pour moduler l'activité d'un récepteur opioïde µ, dans lequel ledit procédé comprend la mise en contact dudit récepteur avec une composition selon la revendication 1.

5. Composition selon l'une quelconque des revendications 1 à 3, destinée à être utilisée dans un procédé pour soulager une douleur qui peut être traitée par une modulation du récepteur opioïde µ, dans laquelle ledit procédé comprend l'administration, à un sujet ayant besoin d'un tel traitement, d'une quantité efficace d'une composition selon la revendication 1.
